# EUROPEAN PATENT APPLICATION

(11) **EP 0 554 643 A1**
(43) Date of publication of application: **11.08.1993**
(21) Application number: 92830049.0
(22) Date of filing: 05.02.1992
(51) Int. Cl.: A61B 3/12, A61B 1/04, A61B 1/26

(54) **High-sensitivity system for examining an object using a low intensity light source**

(71) Applicant: ISTITUTO NAZIONALE DI OTTICA, I-50125 Arcetri Firenze (IT)
(72) Inventor: Longobardi, Giuseppe, I-50125 Firenze (IT); Panzardi, Giuseppe, I-50121 Firenze (IT)
(74) Representative: Mannucci, Gianfranco, Dott.-Ing.

(57) **Abstract**

An equipment for retinal and choroidal angiography comprises means (1) for the illumination of the ocular fundus and means (23,25,27,29) for displaying and/or recording the image of the ocular fundus. Means (19) for image intensification, receive the image information originating from the ocular fundus and send an intensified image to said means for display and/or recording.

## Description

### DESCRIPTION

The invention refers to an apparatus for visualizing an object and/or recording images of said object under low lighting conditions.

In the context of the present invention the word object designates not only an inorganic object, but also and in particular a tissue or an organ of a human or animal being.

More in particular, the invention refers to an apparatus comprising in combination:
- means for generating an electromagnetic radiation;
- first conveying means to send the electromagnetic radiation to the object which has to be observed;
- second conveying means to send the image of said object to displaying and/or recording means.

In many applications the object to be observed cannot be subjected to a high intensity irradiation, since this would damage the object or could cause other problems. This is the case, e.g. in the field of restoration and study of archaeologic objects, such as for example study of papyri.

A first object of the present invention is to provide an apparatus which enables displaying and/or recording of images of objects using a light source, or anyway a source of electromagnetic radiation, having low intensity.

In brief, in a first embodiment the apparatus of the present invention is characterized by means of image intensification, combined to said image displaying and/or recording means, in order to transmit to said display and/or recording means an intensified image of the object. In the study of archaeologic objects, and particularly of papyri, the device according to the invention offers considerable advantages.

More particularly, at present in order to obtain photographic images by means of the fluorescent emission of papyri, it is necessary to expose the papyrus to an UV-radiation for a long time due to the very low intensity of the fluorescent emission of the inks on the papyri. This causes on the one hand a damage of the object under examination, due to the large amount of energy received by the object. On the other hand, the images cannot be obtained in real time, due to the long exposition time and to the subsequent need of developing the photographic films.

The device according to the invention allows to obtain images in very short time at the same intensity of the radiation used for illuminating the object. This also allows to record the images with a television camera system combined to the image intensifier.

Therefore, the device according to the invention allows to perform a new method of examination, characterized in that the image is intensified prior to displaying or recording it. This device and the relevant method can be used in any application wherein the object to be observed must be subjected to an UV-radiation which can cause to the object damages which are directly proportional to the time of exposure. In these cases the use of an image intensifier and of a television camera allows to obtain images in very short time, thus reducing the damage deriving from the UV-radiation to a minimum.

The inventive concept mentioned above has been further developed and improved in order to obtain particular advantages and to solve peculiar problems in the medical field.

In the field of laryngoscopy it is known to use an apparatus comprising an endoscope, a source of light for illuminating the organs to be displayed, first conveying means to send the light emitted by said source to the organs to be illuminated, second conveying means to send the image of said organs to displaying and/or recording means.

Apparatus of this kind are used for studying and examining the vocal chords and in particular for studying their functioning. For this purpose a stroboscopic light source is presently used, which is modulated by the sound vibration generated by the same vocal chords under examination.

These known apparatus have the remarkable disadvantage of a low contrast of the image under examination, which is practically not recordable.

Therefore, a further object of the present invention is to improve an apparatus for endoscopy, and in particular for laryngoscopy, and more particularly for laryngostroboscopy, in order to overcome the disadvantages of the known devices.

In brief, an apparatus for endoscopy, and in particular for laryngoscopy according to the present invention is characterized in that it comprises means of image intensification combined to said displaying and/or recording means in order to transmit to said displaying and/or recording means an intensified image of said organs.

Advantageously, the light source used is a stroboscopic source. In order to allow easy insertion and easy handling of the endoscopy in the larynx, according to a particularly advantageous embodiment of the laryngoscopic apparatus according to the invention provision is made for an image light guide to be combined to the endoscopy, which connects the endoscopy to the displaying means, which may comprise a television camera, a monitor and recording means for recording the detected and intensified image.

The use of an image intensifier in this specific application leads to particular results and advantages. Actually, this makes recording of the images obtained by stroboscopic lighting possible, and in addition this lighting can be very low, so that the dimensions of the endoscope can be remarkably reduced. This renders endoscopy easier and less troublesome to the patient.

Furthermore, in order to obtain specific information about the functioning of the vocal chords, it whould be appropriate to study the vibrations without the patient being hindered by the presence of the endoscope. For this purpose it would be appropriate to introduce the endoscope into the larynx through the nose. According to the state of the art this is made difficult by the dimensions of the endoscope. Moreover, when passing through the nose the endoscope is placed in a position with respect to the vocal chords which is extremely disadvantageous in term of illumination. A consequence of this is that it is practically impossible to obtain significant images through this route with endoscopes of the prior art. The endoscope according to the present invention overcomes these drawbacks since on the one hand it allows to reduce the dimensions of the endoscope, and on the other hand it allows to detect images with a high contrast even under conditions of very low illumination.

Problems related to the need of irradiating an organ with a light radiation in order to obtain an image of the organ for studying and diagnosis purposes arise also in the field of retinal angiography.

A further object of the present invention is to solve the problems related to retinal angiography, which are briefly explained hereinafter.

Fluoroscopic angiography is a medical photography technique which requires fairly sophisticated equipment. This technique is used in the pursuit of the objective of obtaining information for the study and understanding of the circulatory dynamics of the ocular fundus. For this purpose, a small quantity of tracer substance, for example sodium fluorescein, is injected intravenously into the patient. After injection, the fluorescein enters into circulation and reaches the vessels which supply the fundus of the eye. Fluorescent emission by the fluorescein is stimulated by an appropriate method of illumination, in such a way as to make it possible to obtain images which may be recorded on film. Conversely, absorption images may also be recorded. The two techniques, as illustrated in greater detail below, enable different types of information to be obtained.

Photography of the fundus is generally performed with a "fundus camera" capable of analyzing the fundus of the eye and of producing photographic images. The use of a television camera to take television pictures may also be specified. The field of observation is generally illuminated by an intermittent flashing light or by continuous laser light, which in all cases must be sufficiently intense to be capable of exciting a quantity of fluorescein which is kept within very strict limits because of its low tolerability.

An interference filter with a suitably predetermined pass band is interposed between the light source and the fundus of the eye. For retinal fluoroscopic angiography, two filters are normally used: one transmits blue light between 465 and 490 nm, representing the absorption peak of the excitation of fluorescein; the other transmits between 525 and 530 nm, where the emission peak of fluorescein is located.

In order to avoid pseudo-fluorescence (due to the superimposition of of the spectral bands of the filters), filters with a very narrow bandwidth, which are consequently highly selective, have been used. Normal interference filters for a narrow pass band (10 ± 2 nm) have a peak wavelength transmittance of the order of 40-45%, which means that high-power light sources are necessary in order to have, following the interference filter, sufficient intensity to stimulate the emission of the fluorescein.

To enable the structure of the ocular fundus to be displayed, three conditions must be present:
a) the light must reach the structure;
b) the structure must absorb or reflect the light;
c) the adjacent structures must react in a different way from the structure concerned, in order to provide the necessary contrast.

In order to reach the fundus of the eye, the light must pass through the cornea and the crystalline lens, which becomes transparent for light with a wavelength above 320 nm. The various layers of the fundus of the eye can be reached only with certain wavelengths. More particularly, with a wavelength of 460 nm only the anterior retina is reached, while the deep retina is reached with radiation with a wavelength of the order of 593 nm, which penetrates to the pigmented epithelium. Red light radiation with a wavelength of 703 nm reaches the choroid.

With infrared radiation (at approximately 800 nm) it is possible to penetrate the pigmented epithelium of the retina and display the network of the vessels in the choroid. In this case, use is made of the absorption and fluorescence properties of a dye, indocyanine green (ICG), which has an absorption peak at 805 nm and a weak fluorescence with a peak at 835 nm. By comparison with sodium fluorescein, the intensity of fluorescence of indocyanine green (ICG) is thirty times lower. This means that a fluorescence observation at an infrared wavelength requires very high incident luminous intensity and considerable quantities of dye to be injected into the circulatory system of the patient. Consequently, the absorption technique, rather than the fluorescence technique, has been used almost exclusively.

However, as is known to persons skilled in the art, fluoroscopic choroidal angiography yields a body of data much greater than that provided by absorption choroidal angiography. In particular, whereas absorption angiography is sufficient for the diagnosis of tumoral pathologies and for the monitoring of laser treatment in said pathologies, and may be used in the above treatment with IR lasers, fluoroscopic angiography may be used to assess smaller changes in the choroidal vessel network and may be used advantageously for the diagnosis of sub-retinal neovascularizations and for the study of macular pathologies. A single type of equipment is used for both the absorption and the fluoroscopic techniques.

Clearly, therefore, fluoroscopic angiography permits better display of the choroidal vessel network. On the other hand, since the fluorescence signal of indocyanine green is fairly weak, it is necessary to use - in addition to high doses of tracer - very intense illumination (with a power which may exceed 300 W). Moreover, in the case of photographic recording, it is necessary to use particularly sensitive photographic film. The requirement for strong light sources is further increased by the high absorption (up to 70% of the incident intensity) of the interference filters used.

The use of strong light sources entails thermal effects both on the measuring equipment and on the environment, and also on the patient. Means of cooling therefore become necessary, particularly in order to safeguard the interference filters which are particular sensitive to heat. The high power of the light sources, however, remains a cause of serious discomfort for the patient subjected to this type of examination. The most recent types of fluoroscopic angiographs are provided with control means to periodically switch off the light source, thus enabling the equipment to cool down.

The light source (which may be an argon laser with an optical fibre, a flash lamp, or other) is used solely for the excitation of the fluorescence or for absorption examination. In order to position the field of vision correctly and to focus the device with respect to the patient's eye, it is necessary to have a light source of low power, typically 20 W, known as the alignment source. Alignment is carried out by observing the patient's eye through a suitable eyepiece while the light beam from the alignment source is directed against the fundus of the eye. When the correct position has been found, the alignment source is switched off and the high-power light source required to excite the emission of the fluorescent substance is switched on.

The invention relates to a device for retinal or choroidal fluoroscopic angiography or for similar uses, which does not have the typical disadvantages of conventional systems, of which the following may be listed in particular:
- the necessity of high incident luminous intensity on the filters and the resulting necessity of cooling devices, with consequent equipment costs and discomforts for the patient;
- the necessity of injecting high doses of tracer into the patient;
- the difficulty of taking pictures with a television camera;
- the necessity of having a complex optical and illumination system comprising means of alignment and means of illumination.

These disadvantages are overcome with a device of the type specified in the preamble of the attached principal claim, wherein, furthermore, the means of displaying and/or of recording the image are associated with means of image intensification which intensify the image originating from the ocular fundus and send said intensified image to the means of display and/or of recording.

In this way all the above mentioned disadvantages of the conventional systems are overcome. In addition, numerous further advantages are obtained and will be evident to the person skilled in the art from the reading of the following text. In particular, it is possible to produce a device for fluoroscopic angiography which uses a single light source of limited power, for example 20 W electric. This light source may be used both for alignment and for the illumination of the fundus of the eye and the excitation of the tracer. The power supply may be at low voltage. Even when interference filters with high absorption are used, as a result of which the intensity incident on the fundus of the eye is equal, for example, to only 0.7 mW optical, the image intensifier is capable of supplying a sufficiently amplified signal to the means of display to permit detection by a CCD television camera. A monitor associated with the television camera enables the doctor to carry out the alignment and subsequently the observation of the development of the circulatory dynamics of the ocular fundus directly on the display unit, without any need for eyepieces or photographic cameras. The television camera may be associated with means of magnetic recording which, if desired, enable the phenomenon to be viewed again and enable a data archive to be maintained if necessary.

Image intensifiers known as the third-generation type, using a GaAs (gallium arsenide) photocathode, are particularly suitable for application in the present invention. The spectral response of GaAs photocathodes covers the region from 600 to 900 nm (which is the most useful range for the study of the deep retina and of the choroid), where the sensitivity - in other words the ratio between the electrical output current and the intensity of the incident light - is higher than that of a good photocathode of the S20 type fitted on second-generation intensifiers. Between 530 and 600 nm (in other words in the waveband useful for the examination of the anterior retina) the spectral response of GaAs photocathodes is approximately equal to that of the S20 photocathodes of the previous generation. Furthermore, GaAs photocathodes provide a more marked contrast than S20 photocathodes in the near infrared region, thus permitting better fluoroscopic choroidal angiography in this range.

The gain of an image intensifier using a GaAs photocathode may be of the order of 40000, so that it is possible not only to use a light source of very limited power, which therefore causes no discomfort to the patient and does not require special cooling systems or high-wattage power supply units, but also to decrease the quantity of tracer injected into the patient, with obvious advantages from the toxicological viewpoint. The absence of large power supply units and cooling systems also makes it possible to produce portable fluoroscopic angiographs.

Further advantageous embodiments of the device according to the invention are indicated in the attached claims. In particular, the light source may be a light-emitting diode (LED), which emits radiation at a predetermined wavelength, instead of an incoherent light source. In this way the necessity of using an interference filter is eliminated. This is possible because of the fact that with the image intensifier the luminous intensity of the emitting diode is sufficient for making the required observations. By providing a number of diodes used alternately and selectively, it is possible to operate at a number of wavelengths with the same equipment and without the use of interference filters.

The invention also relates to a method comprising the steps of:
- sending a beam of electromagnetic radiation to the object to be examined;
- sending the image of said object to displaying and/or recording means;
- displaying and/or recording said image.

The invention provides for intensifying the image of the object and displaying and/or recording the intensified image.

Further advantageous embodiments of the method according to the invention are indicated in the attached claims.

The invention will be more clearly understood from the description and the attached drawing which shows practical, non-restrictive embodiments of the invention in an application to retinal angiography, an application to laryngoscopy and an application to examination of an inanimate object. In the drawing:
Fig. 1 is a diagram of a possible embodiment of the device for retinal angiography;
Fig. 1A is a diagram of a modified embodiment;
Fig. 2 is a schematic illustration of an image intensifier;
Fig. 3 is a diagram of a device for retinal angiography according to the state of the art;
Fig. 4 is a diagram of an apparatus for laryngoscopy; and
Fig. 5 shows an apparatus for studying an object by means of UV-radiations.

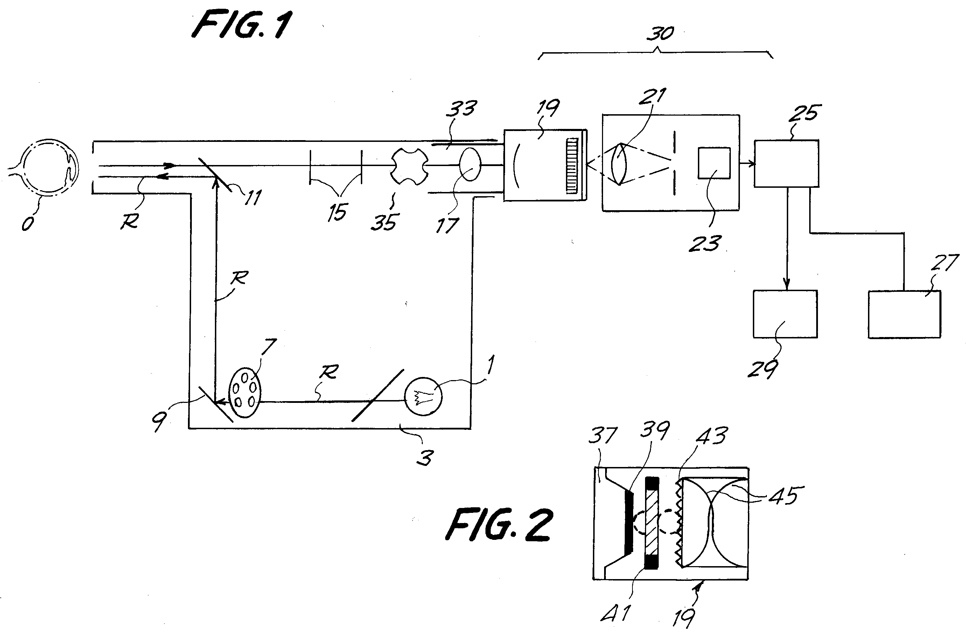

In the embodiment illustrated schematically in Fig. 1, the device comprises a continuous light source 1 of low power, for example 20 W electric, housed in a main housing body 3. The alignment source transmits a light beam R toward a filter carried by a filter support ring 7. The said filter support ring may be fitted with various interference filters to be used for various types of observation. In particular, filters with pass bands centered on the typical absorption and fluorescence wavelengths of the various tracers (fluorescein, indocyanine green) used for observation of the various layers of the fundus of the eye may be disposed on said ring. One of the filters carried by the ring 7 may be used both for alignment and for observation of the fluorescence phenomenon. After the filter support ring 7 there is disposed a pair of deflecting mirrors 9 (reflecting) and 11 (semitransparent), which deflect the beam toward the fundus of the patient's eye O. The patient's eye is kept in the correct position for observation of the region of the fundus of the eye concerned by means of a viewing target which is not illustrated.

With an electric power of 20 W for the light source 1, a continuous luminous intensity of 0.7 mW reaches the fundus of the patient's eye. The electrical power and luminous intensity used are therefore very limited and do not cause any problems either in relation to the thermal effects on the interference filters or in relation to possible discomfort for the patient.

The light beam coming off the fundus of the patient's eye is sent through a set of correcting lenses 15 and a focusing optic 17 toward an image intensifier indicated in general by 19. The image intensifier 19 amplifies the image, with a gain which may be equal to or greater than 40000, and the amplified image is sent through a suitable optic 21 to a CCD television camera 23. The signal from the television camera may then be transmitted through an amplifier 25 to a monitor 27 and/or to means of magnetic recording 29. The viewing section indicated in general by 30, and comprising the focusing optic 17, the image intensifier 19, the optic 21 and the television camera 23, is mounted on a support 33 which is movable with respect to the main housing body 3. A focusing knob 35 enables the viewing section 30 to be moved with respect to the other optical components housed in the main body 3 to permit the focusing of the image.

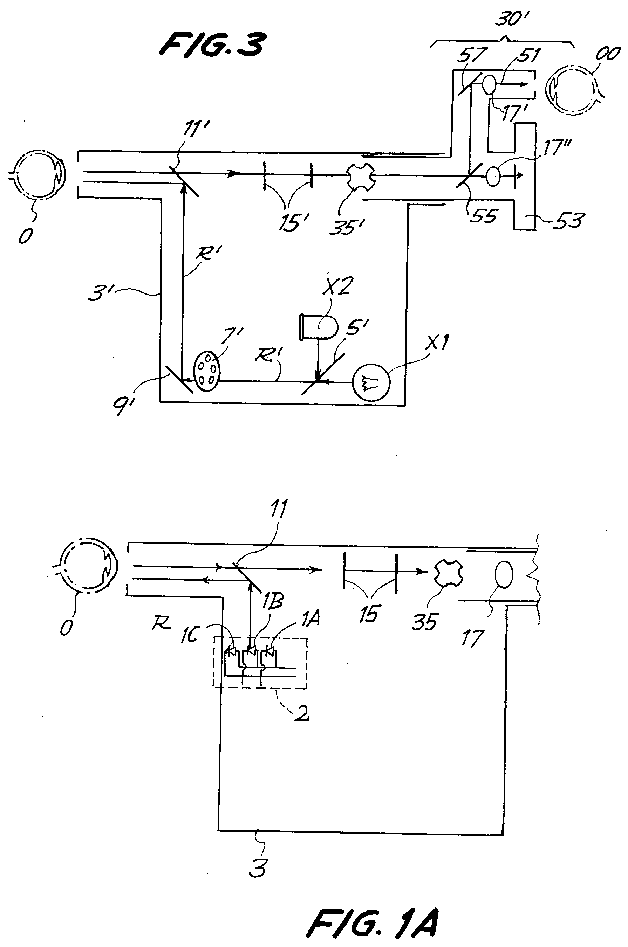

Fig. 2 is a diagram of an image intensifier which may be used as component 19 in the device in Fig. 1. These image intensifiers are known in themselves for other uses. The intensifier in Fig. 3 comprises a glass window 37 through which the incident beam passes. On the rear of the glass window there is deposited a GaAs photocathode 39, which converts the photons of the incident image into electrons. After the photocathode 39 there is disposed an amplifying system 41 for the electrons, consisting of a multichannel plate (MCP). The multichannel plate 41 consists of a very large number of microscopic conducting channels formed in a glass support and disposed in an orderly way in a disk with a thickness of 1 mm. Since each microscopic channel is a separate high-gain electron multiplier, and since there is a one-to-one correspondence between the input face and the output face of the multichannel plate, the multichannel plate intensifies the electronic image. The electrons leaving the multichannel plate 41 are subjected to a very strong electrical field between the plate 41 and a phosphor 43, and are thus "fired" at said phosphor 43, which converts the electrons back to photons which form the intensified optical image. The phosphor 43 is associated with an optical fibre system indicated in general by 45, which is used to re-orientate the inverted image from the focusing optic 17 and to isolate the phosphor 43 from the rest of the equipment following it.

By using an cage intensifier 19 it is possible, as demonstrated above, to use a light source 1 of limited power. In the embodiment in Fig. 1A (in which identical numbers indicate parts identical or corresponding to those of the embodiment in Fig. 1) the light source consists of a set of three light-emitting diodes (LEDs) 1A, 1B, 1C. The light-emitting diodes 1A, 1B, 1C are supported by a movable member 2 indicated by broken lines (for example a slide) in such a way that they can be aligned alternately with the mirror 11. By using LEDs which emit at predetermined wavelengths, it is thus possible to avoid the use of interference filters, further simplifying the structure of the equipment. This is because each LED emits at one of the wavelengths usable for the examination of the fundus of the eye, and the selection of the correct wavelength of the light source is made simply by positioning the correct LED in the optical path of the device. The LEDs which are not in use may be kept switched off if necessary.

Fig. 3 is a diagram of a device for fluoroscopic angiography according to the state of the art. From a comparison of said device with that shown in Fig. 1, and also with the diagram in Fig. 1A, the differences and the advantages obtained with the present invention will become clear. The device in Fig. 3 has a first alignment source of limited power, indicated by X1, which is used to position the light beam with respect to the fundus of the patient's eye O. A second light source X2, which may be continuous or discontinuous (flash) and which may emit incoherent or coherent (laser source) light, of high power (possibly more than 300 W) is used to emit the light beam which, passing through the optical system consisting of the interference filter carried on the ring 7' and the deflecting mirrors 5', 9', 11', is used to excite the fluorescence. 15' indicates a set of correction lenses corresponding to the lenses 15 of the device in Fig.1, while 30' indicates the viewing section which is movable with respect to the main body 3' by means of a focusing knob 35'. The section 30' comprises an eye-piece 51, through which the operator observes the fundus of the patient's eye, and a pair of focusing lenses 17', 17'' corresponding to the lens 17 in Fig. 1. A camera 53 is also provided, to capture images of the fundus of the eye. The image originating from the fundus of the patient's eye is sent to the eyepiece 51 and to the camera 53 by means of a system of mirrors 55 (semi-transparent) and 57 (reflecting). In the schematic representation in Fig.3 the cooling systems and the power supply systems of the two light sources X1, X2 have been omitted.

A comparison of the diagrams in Figs. 1 and 1A and in Fig. 3 makes it clear that the devices in Figs. 1 and 1A have a much simpler structure (without the high-power light source, among other items) and permit much easier display and recording of the images, since all observations, whether during alignment or during the examination of the circulatory dynamics, may be made conveniently through the monitor 27. It will also be noted that the use of a low-power source and of a CCD television camera which captures the image intensified by the intensifier 19 enables television pictures to be taken, making it possible to perform the whole analysis continuously and consequently to gather a quantity of information much greater than that normally possible with the use of discontinuous photography. This is also of particular importance in view of the fact that the tracer injected into the patient's circulatory system takes a very short time to pass through the capillaries of the ocular fundus, so that the time interval in which the examination may be made is very brief. Moreover, the progress in time of the process of re-emission of light by the tracer varies from patient to patient and is therefore unpredictable.

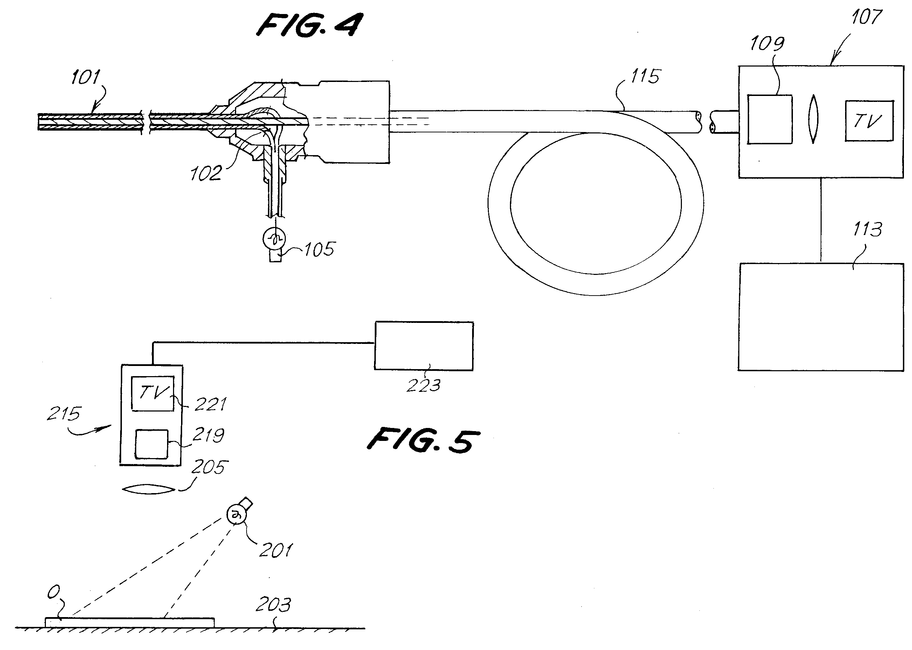

Fig. 4 very diagrammatically shows an apparatus for laryngoscopy, and more particularly an apparatus for laryngostroboscopy using an image intensifying system.

Laryngoscopy is a technique which involves special problems. Actually, a continuous light does not allow the oscillation of the vocal chords to be observed, and in order to examine all the physical properties of the vocal chords, such as frequency, amplitude or phase, stroboscopic systems must be used, i.e. stoboscopic lighting sources.

Irrespect of whether flexible or rigid optic fibre endoscopes are used, the use of stroboscopic illumination (electronically synchronized with the oscillation frequency of the vocal chords) lowers the contrast of the detected image with respect to the image obtained by continuous illumination. This renders vision difficult. The problem is solved by the apparatus of Fig. 4. In this figure, 101 denotes an endoscope of substantially usual type, to which a stroboscopic light source 105 is combined. A bundle of optical fibres 102 conveys the light from source 105 to the tip of endoscope 101. Reference number 107 designates a unit comprising an image intensifier 109, which can be of the type shown in Fig. 2, and a television camera 111. The unit 107 is connected to a monitor and possibly to image recording systems, schematically shown at 113.

The unit 107 is connected to the output of endoscope 101 by means of a flexible image light guide 115 in order to allow for an easier handling of the endoscope during use thereof. Said image light guide 115 has a one-to-one correspondence between the end oriented toward the endoscope 101 output and the end which is in contact with the high sensitivity television system, represented by unit 107. The transmitted image is subjected to a spatial sampling so that the fibre density is sufficiently high in order not to eliminate the higher spatial frequencies. On the other hand, the very limited thickness of the coating allows the contrast at the end coupled to the endoscope to be maintained.

The intensified image coming from the image intensifier 109 provides a high contrast and a high luminosity even if the stroboscopic source 105 has a low or very low intensity. This permits a direct visual observation and also a recording of images of the vocal chords, which images have a high content of information due to the use of the stroboscopic source and which are not affected by the drawback of a low contrast typical of devices of the state of the art.

Fig. 5 is a diagram of an embodiment of the device according to the invention, for use in studying objects by means of UV-radiation, e.g. applicable for studying papyri or other archaeological objects.

In the diagram of Fig. 5, 201 denotes a source of UV-radiation; 203 is a resting plane for the object O to be examined; 205 is a focusing lens; 215 is a unit which comprises an image intensifier 219 (similar to the intensifier 19) and a television camera 221. The television camera 221 is combined to recording and/or displaying means for the detected image, said means are designated 223 as a whole and may include a monitor.

It is to be understood that the drawing illustrates only an example provided solely as a practical demonstration of the invention, it being possible to vary this invention in its forms and dispositions without thereby departing from the scope of the guiding principle of the invention. Any presence of reference numbers in the attached claims has the purpose of facilitating the reading of the claims with reference to the description and to the drawing, and does not limit the scope of protection represented by the claims.

## Claims

1. Equipment for displaying and/or recording images of an object, or part of an object, or of a tissue, organs or parts of a human or animal being, comprising in combination:
- means (1; 1A, 1B, 1C; 105; 201) for generating an electromagnetic radiation to be sent to the object to be observed; and
- conveying means (15, 17; 115; 205) for sending the image of said object to displaying and/or recording means (23, 27; 107, 113; 221, 223);
characterized by:
- means of image intensification (19; 109; 219), combined to said displaying and/or recording means (23, 27; 107, 113; 221, 223) in order to transmit to said displaying and/or recording means an intensified image of said object.

2. Equipment for endoscopy comprising an endoscopy (101), a light source (105) for illuminating the organs to be displayed, first conveying means (102) for sending the light emitted by said source to the organs to be illuminated, second conveying means (115) for sending the image of said organs to displaying and/or recording means (107);
characterized by
means of image intensification (209), combined to said displaying and/or recording means, to send to said displaying and/or recording means an intensified image of said organs.

3. Equipment for retinal and choroidal angiography and similar, comprising means (1; 1A, 1B, 1C) for generating an electromagnetic radiation, which illuminates the ocular fundus, conveying means (15, 17) for sending the image of the ocular fundus to displaying and/or recording means (23, 27);
characterized by:
means for image intensification (19), combined to said displaying and/or recording means, to send an intensified image of the ocular fundus to said displaying and/or recording means.

4. The equipment as claimed in claim 2, wherein the light source is a stroboscopic source.

5. The equipment as claimed in one or more of the preceding claims, wherein the conveying means comprise a flexible image light guide.

6. The equipment as claimed in one or more of the preceding claims, wherein said image intensifier is an intensifier comprising a photocatode of the S20 type.

7. The equipment as claimed in one or more of the preceding claims, wherein said image intensification means comprise a GaAs image intensifier (19).

8. The equipment as claimed in one or more of the preceding claims, wherein the means of illumination comprise a single light source (1) for the alignment and for the examination of the object.

9. The equipment as claimed in claim 8, wherein said light source (1) is a source of incoherent light with a power of less than 100 W.

10. The equipment as claimed in one or more of the preceding claims, comprising at least one light-emitting diode (LED) (1A, 1B, 1C) which emits light at a predetermined wavelength.

11. The equipment as claimed in claim 10, comprising a plurality of light-emitting diodes (LEDs) (1A, 1B, 1C), each of which emits at an operating wavelength of the equipment, the light emitted from one of said diodes being selectively sent toward the fundus of the object.

12. The equipment as claimed in one or more of the preceding claims, comprising a television camera (23; 221) for the capture of the images and possibly a monitor (27; 113, 223) for displaying the image.

13. The equipment as claimed in claim 12, comprising means (29) for recording the image captured by said television camera (23).

14. The equipment as claimed in claim 1, wherein said radiation source (201) is an UV-radiation source.

15. A method for examining and/or recording images of an object, or part of an object, of a tissue, of an organ or part of an organ of a human or animal being, comprising the steps of:
- sending to said object to be examined a beam of electromagnetic radiation;
- sending the image of said object to displaying and/or recording means;
- displaying and/or recording said image;
characterized in that the image of said object is intensified and the intensified image is diaplayed and/or recorded.

16. A method of performing examinations by retinal and choroidal angiography and similar, comprising the phases of sending toward the fundus of the eye a beam of electromagnetic radiation at a predetermined wavelength, capturing the image of the fundus of the eye, and displaying and/or recording said image on suitable means of display and/or of recording, characterized in that the image of the fundus of the eye is intensified before being sent to said means of display and/or of recording.

17. A method for performing an endoscopic examination, comprising the steps of:
- introducing an endoscope in the cavity or in the organ to be examined;
- sending into the cavity or the organ to be examined a beam of electromagnetic radiation;
- detecting the image coming from said cavity or said organ;
- displaying and/or recording said image on suitable displaying and/or recording means;
characterized in that the detected image is intensified before sending it to said displaying and/or recording means.

18. The method as claimed in claim 15, 16 or 17, wherein the detected and intensified image is captured by means of a television camera.

19. The method as claimed in claim 15, wherein said object is illuminated by an UV-radiation.

20. The method as claimed in one or more of claims 16 to 19, wherein a single light source is used to perform the alignment, the focusing and the capturing of the image of the object.
